# EUROPÄISCHE PATENTSCHRIFT

(11) **EP 0 691 861 B1**
(45) Veröffentlichungstag und Bekanntmachung des Hinweises auf die Patenterteilung: **11.11.1998**
(21) Anmeldenummer: 94914263.2
(22) Anmeldetag: 29.03.1994
(51) Int. Cl.: A61M 5/32

(54) **KANÜLENSCHUTZ FÜR EINE INJEKTIONSSPRITZE**
NEEDLE PROTECTOR FOR A SYRINGE
EMBOUT PROTECTEUR POUR SERINGUE D'INJECTION

(30) Priorität: 31.03.1993 AT 656/93; 31.03.1993 AT 657/93
(43) Veröffentlichungstag der Anmeldung: 17.01.1996
(73) Patentinhaber: IMMUNO Aktiengesellschaft, A-1221 Wien (AT)
(72) Erfinder: BARTA, Helmut, A-1100 Wien (AT); MOSER, Franz, A-2232 Deutsch Wagram (AT); SIMONICH, Walter, A-1230 Wien (AT)
(74) Vertreter: Weinzinger, Arnulf, Dipl.-Ing.
(86) Internationale Anmeldenummer: AT9400034
(87) Internationale Veröffentlichungsnummer: WO9422511

(56) Entgegenhaltungen:
- AT-B- 242 286
- DE-C- 848 081
- US-A- 3 889 673
- US-A- 4 850 970

## Beschreibung

Die Erfindung bezieht sich allgemein auf ein verbessertes Injektionsspritzen-System, insbesondere Einwegspritzen-System.

Mehr im einzelnen betrifft die Erfindung einen Kanülenschutz für eine Injektionsspritze, mit einem zur Verbindung mit einem Spritzenkörper vorgesehenen hinteren Befestigungsteil und einem damit einteilig verbundenen, beispielsweise über eine Sollbruchstelle abtrennbaren vorderen Schutzhüllenteil.

Weiters bezieht sich die Erfindung auf einen Kanülen-Modul mit einem solchen Kanülenschutz und einer in einem Kanülenhalter im Inneren des Kanülenschutzes aufgenommenen Kanüle.

Auch betrifft die Erfindung eine Injektionsspritze mit einem Spritzenkörper und einem derartigen Kanülen-Modul, sowie ferner einen Spritzenkörper in einer solchen Injektionsspritze, mit einem Ansatz zur Befestigung eines Kanülen-Moduls.

Schließlich bezieht sich die Erfindung auch auf ein Verfahren zum Montieren und Füllen einer Injektionsspritze.

In der AT-B-242 286 ist ein Kanülenschutz mit einem hinteren Befestigungsteil und einem vorderen Schutzhüllenteil geoffenbart, wobei diese Teile durch eine Sollbruchstelle miteinander verbunden sind. Zur Befestigung der Kanüle ist ein eigener Kanülenhalter vorgesehen, der fest im Kanülenschutz angebracht ist. Zur Befestigung des Kanülenschutzes auf einem Spritzenkörper ist ein Innenkonus im hinteren Befestigungsteil vorgesehen. Bei diesem Kanülenschutz ist jedoch keine Dichtung für die Kanüle vorgesehen; anstattdessen wird der Kanülenschutz insbesondere bei einer Spritze verwendet, bei der eine gesonderte, durch eine Membran abgedichtete Ampulle in einen Zylinder eingesetzt wird, wobei die Membran mittels eines zugespitzten hinteren Kanülenendes durchstochen wird. Das Vorsehen einer eigenen Ampulle, mit einer Membran, sowie eines nach hinten abstehenden zugespitzten Kanülenendes ist jedoch relativ aufwendig.

Aus der US-A -4 735 311, der US-A- 4 986 818 sowie der US-A-5 085 647 sind Einweg-Injektionsspritzen bekannt, bei denen die Kanüle in einem Ansatz des Spritzenkörpers starr montiert und über die Kanüle ein Kanülenschutz bringbar ist, der einen sich zur Kanülenspitze konisch verengten Innenkanal aufweist, der in einen im Bereich der Kanülenspitze erweiterten Hohlraum übergeht. In diesen erweiterten Hohlraum ist ein Gummistopfen einbringbar, in den die Kanülenspitze hineinragt und durch den die vordere Öffnung der Kanüle dicht verschlossen ist. Der Gummistopfen stützt sich kanülenseitig an einer Auflagefläche des Kanülenschutzes ab. Er wird über eine dem Innendurchmesser des Hohlraumes entsprechende Öffnung am vorderen Ende des Kanülenschutzes eingebracht, worauf diese Öffnung durch Verformen des vorderen Endes des Kanülenschutzes derart plastisch umgeformt wird, daß der Gummistopfen im Kanülenschutz unverlierbar gehalten ist. Hierbei verbleibt jedoch am vorderen Ende des Kanülenschutzes eine Öffnung zu dem den Gummipfropfen aufnehmenden Hohlraum frei. Eine vergleichbare Injektionsspritze mit einem zylindrischen Kanülenschutz, in den eine stopfenförmige Kanülendichtung eingeschoben ist, ist aus der DE-C - 848 081 bekannt.

Der Kanülenschutz ist bei diesen bekannten Injektionsspritzen in einfacher Weise auf das vordere Ende des die Kanüle tragenden Teiles des Spritzenkörpers aufgeschoben und rastet gegebenenfalls, beispielsweise gemäß der US-A - 4 735 311, über einen ringförmig vorspringenden Hals des die Kanüle tragenden Teiles des Spritzenkörpers ein. Hierbei ergibt sich das Problem, daß Manipulationen an der Injektionsspritze nicht erkennbar sind, insbesondere ist nicht erkennbar, ob der Kanülenschutz schon einmal entfernt und die Injektionsspritze bereits einmal verwendet wurde.

Bei direkt in den Spritzenkörper eingeklebter Kanüle ergibt sich weiters der Nachteil, daß die Herstellung der Injektionsspritze schwierig ist, weil die Innenwand des Spritzenkörpers in der Regel mit einem Gleitmittel behandelt werden muß, welches bei hoher Temperatur aufgedampft wird. Der Klebstoff, mit dem die Kanüle in den Spritzenkörper eingeklebt ist, hält derart hohe Temperaturen ohne Verfärbung bzw. Zerstörung jedoch nicht aus. Umgekehrt ist es jedoch auch schwierig, einen bereits mit Gleitmittel behandelten Spritzenkörper mit einer Kanüle zu versehen, da eine mit Gleitmittel behandelte Oberfläche einen Klebstoff nicht mehr annimmt.

Die durch eine Klebeverbindung für die Kanüle direkt am Spritzenkörper gegebene Schwierigkeit bei der Behandlung der Innenwand des Spritzenkörpers mit einem Gleitmittel, insbesondere Silikon, (sog. "Silikonisieren" der Spritzenkörper-Innenwand) wird bei der Injektionsspritze nach der AT-B-360 139 u.a. dadurch vermieden, daß die Kanüle in einem Kanülenhalter eingeklebt wird, der nachträglich mittels einer Schnappverbindung auf einen Spritzenkonus des Spritzenkörpers aufgesetzt wird. Zu diesem Zweck ist der Spritzenkonus mit einer peripheren Ringnut ausgebildet, in die haken- oder nasenförmige Rastelemente am hinteren, geschlitzten Ende des Kanülenhalters eingreifen.

Auf den außen konischen Kanülenhalter ist sodann ein Kanülenschutz aufgeschoben.

Die Schnappverbindung für den Kanülennalter am Spritzenkonus ist dabei in zweierlei Hinsicht problematisch: Zum einen soll der Kanülenhalter, im Hinblick auf die eingeklebte Kanüle, aus einem relativ festen, steifen Kunststoffmaterial bestehen, wobei die Rastelemente in der Folge relativ leicht abbrechen können und überdies kaum ein dichter Sitz des Kanülenhalters am Spritzenkonus erzielbar ist, und zum anderen wird die Struktur des Spritzenkonus durch die in ihm eingearbeitete Ringnut geschwächt, so daß der Spritzenkonus relativ leicht abgebrochen werden kann. Ferner kann es zu Rißbildungen im harten Plastikmaterial des Kanülenhalters und damit zu Undichtheiten kommen. Auch bildet hier der Kanülenschutz keine Sicherheit gegen unbefugte Manipulationen, da er nur aufgestülpt ist.

Die US-A-3 889 673 beschreibt einen Kanülenschutz mit einer gesonderten Stirnkappe, die auf einem die Kanüle umgebenden Schutzhüllenteil verrastet ist. Die Zweiteilung des Kanülenschutzes hat dabei den Grund, die Spritze für verschiedene Einsatzzwecke besser geeignet zu machen, wobei einerseits nur die Stirnkappe des Kanülenschutzes abgenommen wird, um durch einen Nippel ein Medikament einer Flüssigkeit in einem Beutel zuzuführen und andererseits der gesamte Kanülenschutz entfernt wird, wenn eine größere Kanülenlänge zum Einführen in eine Medikamentenflasche erforderlich ist. Dabei ist weder eine Abdichtung für den Spritzeninhalt noch eine Überprüfungsmöglichkeit für den Fall von frühzeitigen Manipulationen an der Spritze gegeben, vielmehr ist es problemlos möglich, den gesamten Kanülenschutz oder aber zumindest die Stirnkappe abzunehmen und wieder aufzusetzen, ohne daß dies im nachhinein festgestellt werden kann.

Aus der WO-A-88/00478 ist schließlich eine Injektionsspritze mit Nadelschutzkappe bekannt, bei der der eigentliche Kappenteil mit einem zur Befestigung dienenden Basisteil - über eine Sollbruchstelle - in einem Stück verbunden ist. Die bekannte Nadelschutzkappe ist stirnseitig verschlossen, und sie ist für ein Einwegspritzensystem vorgesehen, bei dem eine scheibenförmige Dichtung am stirnseitigen Ende des Spritzenkörpers selbst vorgesehen ist. Bei Gebrauch der Spritze muß daher zuerst diese Dichtscheibe durch axiales Verstellen der Kanüle nach hinten durchstochen werden, wobei hiefür eine Art Spindeltrieb vorgesehen ist. Eine derartige Ausbildung ist außerordentlich aufwendig und nachteilig hinsichtlich der Fertigung sowie des Zusammenbaus der Kanülen-Modul bzw. der gesamten Injektionsspritze. Darüberhinaus ist von Nachteil, daß bei Inbetriebnahme der Spritze der Kappenteil relativ zum Basisteil und zum Spritzenkörper nur in einer bestimmten Drehrichtung verdreht werden darf, um nach Auftrennen der Sollbruchstelle die Kanüle zum Durchstechen der Dichtscheibe nach hinten zu verstellen. Wenn der Kappenteil irrtümlich in der falschen Drehrichtung verdreht wird, kann es passieren, daß die Kanüle zu weit nach vorne bewegt und die Spritze unbrauchbar wird. Zudem erfordert der Kanülenhalter eine besonders große Länge, da er an seinem vorderen Ende mit einer Verdrehsicherung gegenüber dem Kanülenschutz ausgestattet sein muß. Die Kanüle selbst muß ebenfalls besonders lang ausgebildet sein und muß an beiden Enden zugeschliffen sein. Die doppelseitig zugeschliffene Kanüle ist in den Kanülenhalter eingegossen, was eine komplizierte Fertigung erfordert. Ein Durchstechen der Dichtscheibe kann zur Abtrennung von Teilchen von derselben führen, die dann in den Impfstoff gelangen. In der Folge kann es zu einem teilweisen oder gänzlichen Verstopfen der Kanüle kommen. Ein weiterer Nachteil der bekannten Konstruktion ist darin zu sehen, daß es zu einem erheblichen Druckaufbau des Impfstoffes, z.B. infolge von Temperaturschwankungen, kommen kann, der dann beim Durchstechen der Dichtscheibe zu einem sofortigen Impfstoffaustritt aus der Kanüle führen kann.

Es ist nun eine Aufgabe der Erfindung, die Nachteile der bekannten Systeme zu vermeiden und eine einfache Herstellung sowie insbesondere Montage der einzelnen Komponenten des Spritzensystems zu ermöglichen. Dabei soll insbesondere auch eine vorteilhafte Abdichtung sichergestellt werden können.

Ein weiteres Ziel ist dabei darin gelegen, ein hohes Maß an Sicherheit gegen unbefugte Manipulationen an der Spritze zu ermöglichen.

Ferner ist es Ziel der Erfindung, einen Kanülenschutz bzw. Kanülen-Modul gesondert von der übrigen Spritze fertigen und zusammenbauen zu können,wobei in der Folge auch der Spritzenkörper unabhängig von der Kanüle und ihrer Befestigung mit einem Gleitmittel versehen werden kann, wobei eine streng aseptische Fertigung möglich ist.

Weiters zielt die Erfindung auch darauf ab, eine rationelle, automatische Montage der Spritze sowie deren Befüllung unter sterilen Bedingungen, ohne Kontaminationsgefahr, zu ermöglichen, wobei auch die Möglichkeit geschaffen wird, die jeweilige Flüssigkeit bzw. das Medikament in den Spritzenkörper ohne Zugabe eines Konservierungsmittels einzufüllen.

Der erfindungsgemäße Kanülenschutz der eingangs angegebenen Art ist dadurch gekennzeichnet, daß am offenen vorderen, vom Befestigungsteil abgewandten Ende des Schutzhüllenteils eine als gesonderter Teil vorgesehene, an ihrer vorderen Stirnseite geschlossene Stirnkappe mit einem offenen hinteren Ende befestigt bzw. befestigbar ist, und daß in der Stirnkappe eine stopfenförmige die Kanülenspitze aufnehmende Kanülendichtung aufgenommen ist.

In entsprechender Weise ist der erfindungsgemäße Kanülen-Modul der eingangs angeführten Art dadurch gekennzeichnet, daß die Kanüle stirnseitig über das offene vordere Ende des Schutzhüllenteils vorsteht und in das Innere der Stirnkappe hineinragt, und daß die Kanüle am hinteren Ende fest im Kanülenhalter angebracht ist, der seinerseits fest im hinteren Bereich des Kanülenschutzes sitzt.

Kern der Erfindung ist somit die zweiteilige Ausbildung des Kanülenschutzes, mit dem eigentlichen Schutzhüllenteil in einem Stück mit dem Befestigungsteil einerseits und mit einer daran stirnseitig befestigten bzw. zu befestigenden Stirnkappe andererseits. Durch diese zweiteilige Ausbildung wird nicht nur fertigungstechnisch im Hinblick auf die vereinfachte Herstellung der Komponenten, etwa durch Spritzgießen, ein Vorteil erzielt, sondern überdies auch bei der Montage eine Überprüfung der Anwesenheit und richtigen Lage einer Kanüle vor dem Aufsetzen der Stirnkappe ermöglicht. Von Bedeutung ist ferner das Vorsehen der stopfenförmigen Kanülendichtung in der Stirnkappe, wobei diese Kanülendichtung einfach vor der Anbringung der Stirnkappe am Schutzhüllenteil in dieser Stirnkappe eingesetzt werden kann. In zusammengebauten Zustand sticht die Kanüle mit ihrer Spitze in diese Kanülendichtung ein. Damit kann auch in vorteilhafter Weise eine enge, sich konisch verjüngende Innenbohrung im Kanülenschutz zur Führung der Kanüle beim Aufschieben des Kanülenschutzes über die Kanüle erzielt werden. Als Material für die Kanülendichtung kann dabei in an sich herkömmlicher Weise ein weiches Kautschukmaterial, insbesondere Naturkautschuk, von medizinischer Qualität verwendet werden.

Um einerseits ein einfaches Aufsetzen der Stirnkappe auf den Schutzhüllenteil zu ermöglichen und dabei andererseits ein unbefugtes Abnehmen der Stirnkappe, etwa zu Zwecken einer unerwünschten Manipulation, möglichst hintanzuhalten, ist es sodann von Vorteil, wenn am Schutzhüllenteil und an der Stirnkappe zueinander korrespondierende, für ein Aufsetzen der Stirnkappe auf den Schutzhüllenteil ausgebildete Schnapp- oder Rastverbindungselemente geformt sind.

Aus Gründen einer dichten Verbindung zwischen Stirnkappe und Schutzhüllenteil ist es weiters günstig, wenn der Schutzhüllenteil im Bereich seines vorderen Endes eine stirnseitige Anlagefläche für das hintere Ende der Stirnkappe aufweist. Dabei ist es ferner fertigungstechnisch vorteilhaft, wenn die stirnseitige Anlagefläche des Schutzhüllenteils durch die Stirnfläche eines umlaufenden, einteilig mit dem übrigen Schutzhüllenteil geformten Bundes gebildet ist. Durch die Anlage der Stirnkappe an der stirnseitigen Anlagefläche kann dabei ein bis zu relativ hohen Drücken flüssigkeitsdichter sowie staubdichter Abschluß zwischen den genannten Teilen erzielt werden.

Um ein frühzeitiges Manipulieren am Kanülenschutz bzw. dessen Stirnkappe zu erkennen, wäre es beispielsweise denkbar, an den Stirnkappen-Verbindungselementen eine Sollbruchstelle vorzusehen; andererseits könnte die Verbindung zwischen Stirnkappe und Schutzhüllenteil relativ fest sein, und bei einem Manipulieren an der Stirnkappe könnte dann die zwischen dem Schutzhüllenteil und dem hinteren Befestigungsteil bevorzugt vorgesehene Sollbruchstelle in Funktion treten und brechen, so daß die frühzeitige Manipulation erkennbar wird. Ein besonders hohes Maß an Sicherheit wird jedoch dann erzielt, wenn der Stirnkappe eine eigene Sollbruchstelle im Schutzhüllenteil zugeordnet wird, und es ist demgemäß von besonderem Vorteil, wenn am Schutzhüllenteil benachbart dem vorderen Ende, jedoch im Abstand von der Stelle der Befestigung der Stirnkappe, eine vordere Sollbruchstelle vorgesehen ist. Diese vordere Sollbruchstelle ist dabei zweckmäßigerweise so ausgelegt, daß sie im Falle eines unbefugten Manipulierens an der Stirnkappe bricht, bevor die hintere Sollbruchstelle, zwischen Schutzhüllenteil und Befestigungsteil, aufgetrennt wird.

Die vordere Sollbruchstelle kann einfach dadurch vorgesehen werden, daß der Bund, an dessen vorderer Stirnfläche die Stirnkappe mit ihrem hinteren Ende anliegt, über diese vordere Sollbruchstelle in einen, beispielsweise allgemein zylindrischen, Stirnkappen-Tragteil übergeht.

Die Stirnkappe sowie der übrige Kanülenschutz bestehen vorzugsweise aus einem relativ weichen Kunststoffmaterial, wie etwa einem Polyethylen, wobei sich ein einrastendes Aufdrücken der Stirnkappe auf den Schutzhüllenteil bzw. gegebenenfalls dessen Stirnkappen-Tragteil auch dann als möglich erweist, wenn die Stirnkappe an ihrem hinteren Ende in Umfangsrichtung geschlossen ist, d.h. keine achsparallelen Längsschlitze aufweist. In diesem Zusammenhang kann die gewünschte rasch herbeizuführende und danach praktisch nicht mehr zu lösende Rastverbindung in besonders vorteilhafter Weise dadurch erzielt werden, daß am vorderen Ende des Schutzhüllenteils, gegebenenfalls am Stirnkappen-Tragteil, eine ringförmige Rastnut gebildet ist, die mit einem am hinteren Ende der Stirnkappe vorgesehenen, radial einwärts vorstehenden Rastvorsprung zur unlösbaren Befestigung der Stirnkappe am Schutzhüllenteil zusammenarbeitet.

Im Hinblick auf eine leicht herbeizuführende, dann jedoch feste und dichte, zuverlässige Verbindung zwischen Kanülenschutz und Kanülenhalter hat es sich als vorteilhaft gezeigt, wenn der Befestigungsteil einen Innenkonus sowie eine ringförmige Nut zur Aufnahme eines mit einem korrespondierenden Außenkonus und einem korrespondierenden peripher umlaufenden ringförmigen Vorsprung ausgestatteten Kanülenhalters aufweist.

Für einen sicheren, dichten Halt des Kanülenschutzes auf dem Spritzenkörper hat es sich weiters als günstig erwiesen, wenn im hinteren Befestigungsteil innen eine zu einem Ringwulst an einem z.B. konusförmigen Ansatz des Spritzenkörpers passende Rastnut vorgesehen ist. Dabei wird ein weiterer Vorteil dadurch erzielt, daß der beispielsweise aus Borsilikat-Glas bestehende Spritzenkörper im Bereich seines Ansatzes ungeschwächt ist, da als Rastelement dort ein Ringwulst, anstatt wie bei früheren Spritzen eine periphere Ringnut, vorgesehen ist.

Was den erfindungsgemäßen Kanülen-Modul und dabei insbesondere die Befestigung der Kanüle im Kanülenhalter anlangt, so kann beim vorliegenden System die Kanüle einfach im Kanülenhalter eingeklebt sein. Der Kanülenhalter kann dabei problemlos aus einem relativ starren, steifen Material, wie etwa einem Cellulosepropionat, bestehen, wobei er vom relativ weichen Material, etwa Polyethylen, des Kanülenschutzes umschlossen ist, um so am Spritzenkörper mittelbar, über den Befestigungsteil des Kanülenschutzes, befestigt zu werden. Zum Einkleben der Kanüle im Kanülenhalter wird ein an sich herkömmlicher medizinischer Kleber eingesetzt, und die Kanüle kann beispielsweise aus einem Chrom-Nickel-Stahl bestehen.

Für ein einfaches Einkleben der Kanüle unter Gewährleistung eines sicheren Halts im Kanülenhalter hat es sich weiters als günstig erwiesen, wenn der Kanülenhalter am vorderen Stirnende eine sich konisch erweiternde Klebstoffaufnahme aufweist. Auf diese Weise wird das Einkleben der Kanüle im Kanülenhalter erleichtert, wobei die Kanüle nichstdestoweniger einen ausreichend festen Sitz im Kanülenhalter erhält, auch wenn sie nicht über ihre gesamte Länge im Inneren des Kanülenhalters mit letzterem verklebt ist.

Ein besonderer Gedanke bei der vorliegenden Erfindung ist darin gelegen, daß die Befestigung der Kanüle am Spritzenkörper möglichst starr und unentfernbar gestaltet ist, wobei jedoch trotz Verwendung eines entsprechend starr ausgebildeten Kanülenhalters - in dem die Kanüle in besonders einfacher Art und Weise einklebbar ist und einen für die Verwendung ausreichend guten Halt findet - ein einfaches Ansetzen der Kanüle an dem Spritzenkörper ohne jede Beschädigung des Kanülenhalters sichergestellt ist. In diesem Zusammenhang ist ein fester, dichter Sitz des Kanülenhalters im Kanülenschutz von Bedeutung, und es ist hier von Vorteil, wenn der Kanülenhalter in dem die Kanüle umgebenden Kanülenschutz starr eingesetzt ist, wobei der Kanülenhalter einen Außenkonus und der Kanülenschutz einen zu dem Außenkonus des Kanülenhalters korrespondierenden Innenkonus aufweist. Weiters ist es auch günstig, wenn am Außenkonus des Kanülenhalters an dessem dem Spritzenkörper zugewendeten Ende ein peripher umlaufender ringförmiger Vorsprung vorgesehen ist und der Kanülenschutz am Innenkonus eine zu diesem Vorsprung korrespondierende Nut aufweist.

Um bei Inbetriebnahme der Injektionsspritze das Abtrennen des Schutzhüllenteils des Kanülenschutzes, insbesondere durch Verdrehen in der einen oder anderen Richtung, zu erleichtern, ist es ferner von Vorteil, wenn die vordere Stirnseite des Kanülenhalters etwas vor der das Abtrennen des Schutzhüllenteils ermöglichenden Sollbruchstelle gelegen ist.

Gemäß einem weiteren Aspekt betrifft die vorliegende Erfindung auch eine Injektionsspritze mit einem Spritzenkörper und einem erfindungsgemäßen Kanülen-Modul sowie weiters einen Spritzenkörper in einer solchen Injektionsspritze, mit einem Ansatz zur Befestigung eines erfindungsgemäßen Kanülen-Moduls. Dabei ergibt sich bereits aus den vorstehenden Erläuterungen des vorliegenden Spritzensystems, daß der Kanülenhalter des Kanülen-Moduls nicht direkt, sondern indirekt, über den Befestigungsteil des Kanülenschutzes, mit dem Spritzenkörper zu verbinden ist. Demgemäß ist die erfindungsgemäße Injektionsspritze dadurch gekennzeichnet, daß der Kanülen-Modul unter Anordnung des Kanülenhalters unmittelbar vor einem am Spritzenkörper vorgesehenen Spritzenkonus mit dem Befestigungsteil seines Kanülenschutzes auf diesen Spritzenkonus aufgesetzt ist. Hierbei ist es weiters von Vorteil, wenn der Kanülen-Modul mit dem Befestigungsteil auf dem Spritzenkörper verrastet ist, wobei ein peripherer Ringwulst am Spritzenkonus in eine Ringnut in der Innenwand des Befestigungsteils eingreift. Auch ist es hier günstig, wenn der Spritzenkonus des Spritzenkörpers an seinem hinteren Ende eine an den Ringwulst anschließende periphere rundumlaufende und gegenüber dem Ringwulst und vorzugsweise auch gegenüber dem Spritzenkonus rückspringende Nut aufweist, wobei der Kanülenschutz unter Bildung einer an sich bekannten Schnapp- oder Rastverbindung korrespondierend hierzu ausgestaltet ist. Ferner ist es auch von Vorteil, wenn die Außenfläche des Spritzenkonus, gegebenenfalls teilweise, aufgerauht ist, um einem Verdrehen des aufgesetzten Befestigungsteils beim Abtrennen des Schutzhüllenteils durch dessen Verdrehen entgegenzuwirken. Die vom Spritzenkörper getrennte Befestigung der Kanüle in einem Kanülenhalter ermöglicht es, daß der Spritzenkörper aus einem Glaskörper besteht, dessen mit einem Gleitmittel versehene Innenwandung bei hoher Temperatur behandelt ist, vorteilhaft mit Silikon bei etwa 300°C.

Der erfindungsgemäße Spritzenkörper ist in entsprechender Weise dadurch gekennzeichnet, daß als Ansatz ein mit einem peripheren Ringwulst versehener Spritzenkonus vorgesehen ist.

In entsprechender Weise ist es auch von Vorteil, wenn als Ansatz ein mit einer, gegebenenfalls teilweise, aufgerauhten Außenfläche versehener Spritzenkonus vorgesehen ist.

Für den besonders festen Halt des Kanülenmoduls ist es ferner günstig, wenn der Spritzenkonus an seinem hinteren Ende einen peripher umlaufenden Ringwulst und eine an den Ringwulst anschließende, peripher umlaufende, ringförmige Nut aufweist.

Das vorliegende Spritzensystem ermöglicht in vorteilhafter Weise eine aseptische Fertigung und maschinelle Verarbeitung, insbesondere in Form einer sog. "in-line"-Montage und -Befüllung, wobei manuelle Eingriffe und eine etwaige Kontaminationsgefahr vermieden werden können. Demgemäß zielt die Erfindung auch auf ein vorteilhaftes Verfahren zum Montieren und Befüllen einer Injektionsspritze gemäß der Erfindung ab, und dieses Verfahren ist dadurch gekennzeichnet, daß der Spritzenkörper gesondert innen silikonisiert und anschließend sterilisiert wird, daß unabhängig davon ein sterilisierter Kanülen-Modul durch Einkleben der Kanüle im Kanülenhalter, Einsetzen des Kanülenhalters samt Kanüle in den Kanülenschutz sowie Aufsetzen der mit der stopfenförmigen Kanülendichtung versehenen Stirnkappe auf den Schutzhüllenteil, nach Prüfung auf Vorliegen einer Kanüle in richtige Lage am offenen Ende des Schutzhüllenteils, zusammengebaut wird, und daS der Kanülen-Modul unter sterilen Bedingungen auf den Spritzenkörper dicht aufgesetzt und schließlich die Spitze befüllt sowie durch Einsetzen eines Kolbenstopfens am hinteren Ende verschlossen wird. Die Teile der Kanülen-Modul werden dabei zweckmäßigerweise strahlensterilisiert.

Die Erfindung wird nachstehend anhand eines in der Zeichnung veranschaulichten, besonders bevorzugten Ausführungsbeispiels, auf das sie jedoch nicht beschränkt sein soll, noch weiter erläutert.

Im einzelnen zeigen:

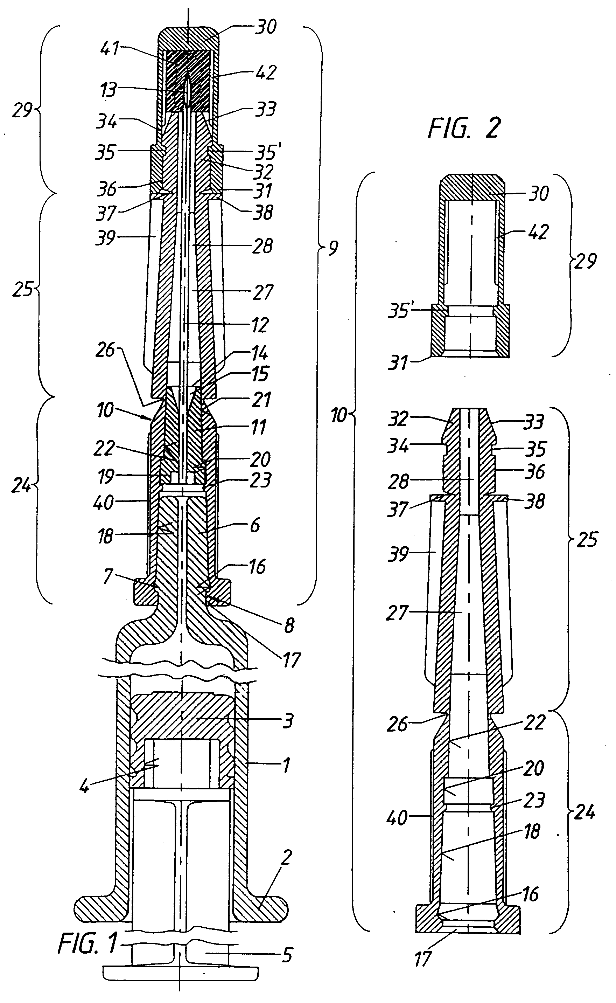

Fig.1 einen Längsschnitt durch eine Einweg-Injektionsspritze; und
Fig.2 einen zugehörigen Kanülenschutz in Explosionsdarstellung, ebenfalls im Längsschnitt.

Mit 1 ist ein zylindrischer, aus Glas gefertigter Spritzenkörper bezeichnet, der an seinem hinteren Ende einen Flansch 2 aufweist. Der Spritzenkörper 1 ist an diesem Ende durch einen Kolbenstopfen 3 verschließbar, der eine Ausnehmung 4 aufweist, in die eine Betätigungsstange 5 einsetzbar ist. An seinem vorderen Ende endigt der Spritzenkörper 1 in einem Spritzenkonus 6, der an seinem hinteren Ende einen peripher geringfügig vorragenden umlaufenden Ringwulst 7 und eine an den Ringwulst 7 anschließende peripher umlaufende ringförmige Nut 8 aufweist. Der Spritzenkörper 1 weist an der Stelle der ringförmigen Nut 8 einen kleineren Durchmesser auf als der Spritzenkonus 6 an seiner dicksten Stelle. Der Durchmesserunterschied ist jedoch nur gering bemessen.

Auf dem Spritzenkonus 6 ist ein Kanülen-Modul 9 aufgesetzt, der als eigene Baueinheit unabhängig vom Spritzenkörper 1 hergestellt ist. Dieser Kanülen-Modul 9 weist einen Kanülenschutz, der generell mit 10 bezeichnet ist, auf, in dem ein Kanülenhalter 11 starr eingesetzt ist. Eine Kanüle 12 ist in dem Kanülenhalter 11 eingeklebt, zu welchem Zweck der Kanülenhalter 11 an seinem der Kanülenspitze 13 zugewandten Ende eine sich zur Kanülenspitze 13 erweiternde trichterförmige Ausnehmung 14 aufweist, die zur Aufnahme eines Klebstoffes 15 dient. Als Klebstoff wird vorteilhaft ein für medizinische Zwecke geeigneter Kleber eingesetzt.

Der Kanülenhalter 11 selbst ist aus einem relativ starren, steifen Kunststoff, wie beispielsweise Cellulosepropionat, gebildet; die Kanüle 12 ist beispielsweise aus Chrom-Nickel-Stahl gefertigt. Der Kanülenschutz 10 selbst besteht aus relativ weichem Kunststoffmaterial, wie beispielsweise Polyethylen oder Polypropylen. Er läßt sich daher in relativ einfacher Weise über den Spritzenkonus 6 und dessen am hinteren Ende angeordneten Ringwulst 7 schieben und mittels einer korrespondierend zu dem Ringwulst 7 ausgebildeten ringförmigen Nut 16 am Ringwulst 7 einschnappend befestigen. Am hinteren Ende ist der Kanülenschutz 10 mit einem Einlaufkonus 17 versehen, der das geringfügige Aufweiten des Kanülenschutzes 10 während des Aufsetzvorganges auf den Spritzenkonus 6 erleichtert.

Eine Dichtheit zwischen dem Spritzenkonus 6 und dem hinteren Ende des Kanülenschutzes 10 wird dadurch erreicht, daß der Kanülenschutz 10 einen korrespondierend zum Spritzenkonus 6 ausgebildeten Innenkonus 18 aufweist. Der Kanülenhalter 11 ist knapp vor dem Ende des Spritzenkonus 6 im Kanülenschutz 10 verankert, u.zw. durch einen ringförmigen, am hinteren Ende des Kanülenhalters 11 angeordneten, radial vorspringenden Bund 19, der in eine korrespondierend zu diesem Bund 19 angeordnete Nut 20 des Kanülenschutzes 10 eingreift. Auch hier wird eine Dichtheit zwischen dem Kanülenschutz 10 und dem Kanülenhalter 11 dadurch erzielt, daß der Kanülenhalter 11 einen Außenkonus 21 und der Kanülenschutz 10 einen korrespondierend dazu angeordneten Innenkonus 22 aufweist.

Die den Bund 19 aufnehmende Nut 20 ist an ihrem dem Spritzenkonus 6 zugewandten Ende durch einen kleinen, radial nach innen vorstehenden Ringwulst 23 abgeschlossen, so daß der Kanülenhalter 11 nach dem Einpressen in den Kanülenschutz 10 in axialer Richtung ausreichend fixiert ist.

Der Kanülenschutz 10 ist teilweise von einem Befestigungsteil 24 gebildet, der die Stützfunktion für den Kanülenhalter 11 und auch die Stützfunktion hinsichtlich der Befestigung am Spritzenkörper 1 übernimmt. Integral mit diesem Befestigungsteil 24 ist ein Schutzhüllenteil 25 ausgebildet, wobei diese beiden Teile durch eine soll-Bruchstelle 26 miteinander in Verbindung stehen. Diese Soll-Bruchstelle 26 wird durch eine extreme Wandstärkenverdünnung gebildet, die knapp hinter dem vorderen Ende des Kanülenhalters 11 zu liegen kommt. Der Schutzhüllenteil 25 weist einen sich konisch verjüngenden Innenraum 27 auf, der in Richtung Kanülenspitze 13 in einen zylindrischen Innenraum 28 übergeht. Er ist etwas kürzer gehalten als die Länge der Kanüle 12, so daß die Kanülenspitze 13 mit ihrem schrägen Anschliff zur Gänze außerhalb des Schutzhüllenteiles 25 zu liegen kommt.

Zum Kanülenschutz 10 gehört noch eine Stirnkappe 29, die eine vordere geschlossene Stirnseite 30 und ein offenes hinteres Ende 31 aufweist, mit dem sie über den Endteil 32 des Schutzhüllenteiles 25 schiebbar ist. Dieser Endteil 32 weist von seinem vorderen Ende aus gesehen einen Auflaufkegel 33 auf, dem ein kurzer zylindrischer Abschnitt 34 folgt. Dieser geht in eine ringförmige Nut 35 über, in die ein ringförmiger Innenvorsprung 35' der Stirnkappe 29 einrastbar ist. Dieser Nut 35 folgt ein zylindrischer Führungsteil 36, der das Aufsetzen der Stirnkappe 29 erleichtert.

Dieser zylindrische Führungsteil 36 geht über eine Soll-Bruchstelle 37, die ebenfalls von einer ringförmig umlaufenden Wandengstelle gebildet ist, in einen sich radial nach außen erstreckenden Bund 38 über. Von dem Bund 38 ausgehend erstrecken sich bis nahe zur beim Kanülenhalter 11 angeordneten Soll-Bruchstelle 26 reichende, nach außen vorstehende Griffrippen 39, die ein Abdrehen des Schutzhüllenteiles 25 vom Befestigungsteil 24 erleichtern. Der Befestigungsteil 24 braucht hierbei nicht gehalten werden; er weist jedoch ebenfalls vorstehende Längsrippen 40 auf, die die Griffigkeit erhöhen. Durch seinen konischen Klemmsitz am Spritzenkörper 1 hat der Befestigungsteil 24 einen genügenden Halt, so daß ein Mitdrehen desselben beim Entfernen des Schutzhüllenteiles 25, also ein Drehen des Befestigungsteiles 24 gegenüber dem Spritzenkörper 1, verhindert ist. Durch ein Aufrauhen der Außenseite des Spritzenkonus 6 kann der Halt des Befestigungsteiles 24 am Spritzenkonus 6 noch weiter verbessert werden.

In die Stirnkappe 29 ist eine Kanülendichtung 41 eingesetzt, in die die Spitze 13 der Kanüle 12 einstechbar ist, so daß die Kanüle 12 an ihrem vorderen Ende verschlossen ist. Diese Kanülendichtung 41 ist vorzugsweise aus Naturkautschuk gefertigt. Sie wird vor Aufbringen der Stirnkappe 29 auf den Schutzhüllenteil 25 in die Stirnkappe 29 eingesetzt und hält in dieser infolge innenseitig angeordneter, in radialer Richtung nach innen vorragender Rippen 42.

Wie aus der Zeichnung zu erkennen ist, weist die Stirnkappe 29 eine solche Länge auf, daß ihr offenes Ende 31 an dem umlaufenden Bund 38 des Schutzhüllenteiles 25 ansteht. Die Größe der Kanülendichtung 41 ist derart bemessen, daß bei auf dem Schutzhüllenteil 25 aufgesetzter Stirnkappe 29 eine leichte Quetschung der Kanülendichtung 41 stattfindet, so daß eine absolute Dichtheit des vom Schutzhüllenteil 25 eingeschlossenen Hohlraumes 27, 28 gegenüber der Außenatmosphäre gegeben ist.

Die ringförmige Nut 35 des einen Stirnkappen-Teil bildenden Endteiles 32 des Kanülenschutzes 10 und der dazu korrespondierende ringförmige Innenvorsprung 35' der Stirnkappe 29 sind derart gestaltet, daß ein Einrasten der Stirnkappe 29 ohne Schwierigkeit durchführbar ist, daß jedoch ein Lösen nicht mehr möglich ist. Sollte man ein Lösen dennoch versuchen, kommt es zu einem Aufreißen der vorderen Soll-Bruchstelle 37. Versucht man, nach dem Aufreißen der vorderen Soll-Bruchstelle 37 die Stirnkappe 29 wieder auf die Kanüle 12 aufzusetzen, so kommt es zu einem geringfügigen Abfedern der Stirnkappe 29 (und des in ihr befindlichen Endteiles 32 des Schutzhüllenteiles 25) vom Bund 38 des Schutzhüllenteiles 25 infolge der federnden Eigenschaften der Kanülendichtung 41. Zwischen dem Ende 31 der Stirnkappe 29 und dem Bund 38 ist somit sofort ein Zwischenraum erkennbar, und der Benützer weiß, daß die Stirnkappe 29 schon einmal entfernt wurde, daß also eine Manipulation stattgefunden hat.

Der Vorteil der oben beschriebenen Konstruktion liegt darin, daß die Kanüle 12 zur Gänze von Bauteilen umgeben ist, die sich von der Kanüle 12 nicht entfernen lassen, ohne daß nicht sofort eine derartige Manipulation erkennbar wäre. Wesentlich ist auch, daß die Kanülendichtung 41 zur Gänze nach außen geschützt untergebracht ist, so daß Manipulationen an der Kanülendichtung 41 ebenfalls nicht möglich sind, ohne daß man sie sofort von außen erkennt.

Eine maschinelle Fertigung des Kanülen-Moduls 9 läßt sich zweckmäßig wie folgt durchführen:

Zunächst werden die beiden Teile des Kanülenschutzes 10, also die Stirnkappe 29 einerseits und der mit dem Befestigungsteil 24 integral zusammenhängende Schutzhüllenteil 25, vorteilhafterweise unter entsprechenden Reinraumbedingungen, im Spritzgußverfahren hergestellt. In einem auf gleiche Weise hergestellten Kanülenhalter 11 wird auf einer eigenen Montagemaschine die Kanüle 12 mit einem entsprechenden Kleber eingeklebt. Sodann wird der Kanülenhalter 11 mit der Kanüle 12 in den Kanülenschutz 10 eingedrückt, bis die vorgesehene Konus-Dichtverbindung bei 21, 22 erzielt ist.

Am vorderen Ende des Schutzhüllenteiles 24 ragt die Kanüle 12 mit ihrer zugeschliffenen Spitze 13 hervor. Es erfolgt anschließend ein zweckmäßig optisches Prüfen des Vorhandenseins einer Kanüle 12 sowie eine Prüfung, ob die Kanüle 12 ihre richtige Lage - Spitze 13 vorne - einnimmt. Wird festgestellt, daß die Kanüle nicht mit dem Anschliff in der richtigen Position vorhanden ist, wird der Teil als schlecht ausgeschieden. Sodann erfolgt die Durchgangsprüfung der Kanüle 12. Anschließend wird die Stirnkappe 29 mit der vorher in sie eingedrückten Kanülendichtung 41 auf den Endteil 32 aufgedrückt, bis die Schnappverbindung (35, 35') einrastet. Sonach erfolgt das Einschweißen des Kanülen-Moduls 9 in eine Verpackung, und es kann nachfolgend ein Strahlensterilisieren durchgeführt werden.

Unabhängig von dieser Fertigung erfolgt das Herstellen der Spritzenkörper 1 aus Glas. Diese werden in Trays angeliefert und maschinell aus den Trays herausgeholt. Nach einem Waschen werden die Spritzenkörper 1 innen silikonisiert. Anschließend werden sie in Magazine eingelegt und in diesen durch einen Sterilisier tunnel (etwa 300°C) gefahren.

Nach dem Sterilisiervorgang werden die Spritzenkörper 1 aus den Magazinen herausgenommen und einer Kanülen-Modul-Aufdrückstation zugeführt. Die strahlensterilisierten Kanülen-Module 9 werden auf die Spritzenkörper 1 einrastend aufgedrückt, wodurch eine dichte Verbindung zwischen diesen beiden Teilen gegeben ist.

In einer folgenden Station wird der Spritzenkörper 1 befüllt, worauf der Kolben 3 eingesetzt wird. In diesem Zustand ist die Einweg-Injektionsspritze bereits dicht verschlossen. Sie wird über ein Transportsystem aus dem Sterilbereich befördert und wieder in die Trays eingelegt.

## Patentansprüche

1. Kanülenschutz (10) für eine Injektionsspritze, mit einem zur Verbindung mit einem Spritzenkörper (1) vorgesehenen hinteren Befestigungsteil (24) und einem damit einteilig verbundenen, beispielsweise über eine Sollbruchstelle (26) abtrennbaren vorderen Schutzhüllenteil (25), dadurch gekennzeichnet, daß am offenen vorderen, vom Befestigungsteil (24) abgewandten Ende des Schutzhüllenteils (25) eine als gesonderter Teil vorgesehene, an ihrer vorderen Stirnseite (30) geschlossene Stirnkappe (29) mit einem offenen hinteren Ende (31) befestigbar ist, und daß in der Stirnkappe (29) eine stopfenförmige, die Kanülenspitze (13) aufnehmende Kanülendichtung (41) aufgenommen ist.

2. Kanülenschutz nach Anspruch 1, dadurch gekennzeichnet, daß am Schutzhüllenteil (25) und an der Stirnkappe (29) zueinander korrespondierende, für ein Aufsetzen der Stirnkappe (29) auf den Schutzhüllenteil (25) ausgebildete Schnapp- oder Rastverbindungselemente (35, 35', 36) geformt sind.

3. Kanülenschutz nach Anspruch 1 oder 2, dadurch gekennzeichnet, daß der Schutzhüllenteil (25) im Bereich seines vorderen Endes eine stirnseitige Anlagefläche für das hintere Ende (31) der Stirnkappe (29) aufweist.

4. Kanülenschutz nach Anspruch 3, dadurch gekennzeichnet, daß die stirnseitige Anlagefläche des Schutzhüllenteils (25) durch die Stirnfläche eines umlaufenden, einteilig mit dem übrigen Schutzhüllenteil (25) geformten Bundes (38) gebildet ist.

5. Kanülenschutz nach einem der Ansprüche 1 bis 4, dadurch gekennzeichnet, daß am Schutzhüllenteil (25) benachbart dem vorderen Ende, jedoch im Abstand von der Stelle der Befestigung der Stirnkappe (29), eine vordere Sollbruchstelle (37) vorgesehen ist.

6. Kanülenschutz nach Anspruch 4 und 5, dadurch gekennzeichnet, daß der Bund (38) über die vordere Sollbruchstelle (37) in einen Stirnkappen-Tragteil (32) übergeht.

7. Kanülenschutz nach einem der Ansprüche 2 bis 6, dadurch gekennzeichnet, daß am vorderen Ende des Schutzhüllenteils (25) eine ringförmige Rastnut (35) gebildet ist, die mit einem am hinteren Ende der Stirnkappe (29) vorgesehenen, radial einwärts vorstehenden Rastvorsprung (35') zur unlösbaren Befestigung der Stirnkappe (29) am Schutzhüllenteil (25) zusammenarbeitet.

8. Kanülenschutz nach einem der Ansprüche 1 bis 7, dadurch gekennzeichnet, daß der Befestigungsteil (24) einen Innenkonus (22) sowie eine ringförmige Nut (20) zur Aufnahme eines mit einem korrespondierenden Außenkonus (21) und einem korrespondierenden peripher umlaufenden ringförmigen Vorsprung ausgestatteten Kanülenhalters (11) aufweist.

9. Kanülenschutz nach einem der Ansprüche 1 bis 8, dadurch gekennzeichnet, daß im hinteren Befestigungsteil (24) innen eine zu einem Ringwulst (7) an einem z.B. konusförmigen Ansatz (6) des Spritzenkörpers (1) passende Rastnut (16) vorgesehen ist.

10. Kanülen-Modul (9) mit einem Kanülenschutz (10) gemäß einem der Ansprüche 1 bis 9 und einer in einem Kanülenhalter (11) im Inneren des Kanülenschutzes aufgenommenen Kanüle (12), dadurch gekennzeichnet, daß die Kanüle (12) stirnseitig über das offene vordere Ende des Schutzhüllenteils (25) vorsteht und in das Innere der Stirnkappe (29) hineinragt, und daß die Kanüle (12) am hinteren Ende fest im Kanülenhalter (11) angebracht ist, der seinerseits fest im hinteren Bereich des Kanülenschutzes (10) sitzt.

11. Kanülen-Modul nach Anspruch 10, dadurch gekennzeichnet, daß die Kanüle (12) im Kanülenhalter (11) eingeklebt ist.

12. Kanülen-Modul nach Anspruch 11, dadurch gekennzeichnet, daß der Kanülenhalter (11) am vorderen Stirnende eine sich konisch erweiternde Klebstoffaufnahme (14) aufweist.

13. Kanülen-Modul nach einem der Ansprüche 10 bis 12, dadurch gekennzeichnet, daß der Kanülenhalter (11) in dem die Kanüle (12) umgebenden Kanülenschutz (10) starr eingesetzt ist, wobei der Kanülenhalter (11) einen Außenkonus (21) und der Kanülenschutz (10) einen zu dem Außenkonus (21) des Kanülenhalters (11) korrespondierenden Innenkonus (22) aufweist.

14. Kanülen-Modul nach einem der Ansprüche 10 bis 12, dadurch gekennzeichnet, daß die vordere Stirnseite des Kanülenhalters (11) etwas vor der das Abtrennen des Schutzhüllenteils (25) ermöglichenden Sollbruchstelle (26) gelegen ist.

15. Kanülen-Modul nach einem der Ansprüche 10 bis 14, dadurch gekennzeichnet, daß der Kanülenhalter (11) aus einem harten Kunststoffmaterial, beispielsweise aus Cellulosepropionat, gebildet ist, und daß der Kanülenschutz (10) aus einem weicheren Kunststoffmaterial, beispielsweise aus Polyethylen oder Polypropylen, gebildet ist.

16. Kanülen-Modul nach einem der Ansprüche 13 bis 15, dadurch gekennzeichnet, daß am Außenkonus (21) das Kanülenhalters (11) an dessem dem Spritzenkörper (1) zugewendeten Ende ein peripher umlaufender ringförmiger Vorsprung (19) vorgesehen ist und der Kanülenschutz (10) am Innenkonus (22) eine zu diesem Vorsprung (19) korrespondierende Nut (20) aufweist.

17. Injektionsspritze mit einem Spritzenkörper (1) und einem Kanülen-Modul (9) nach einem der Ansprüche 10 bis 16, dadurch gekennzeichnet, daß der Kanülen-Modul (9) unter Anordnung des Kanülenhalters (11) unmittelbar vor einem am Spritzenkörper (1) vorgesehenen Spritzenkonus (6) mit dem Befestigungsteil (24) seines Kanülenschutzes (10) auf diesen Spritzenkonus (6) aufgesetzt ist.

18. Injektionsspritze nach Anspruch 17, dadurch gekennzeichnet, daß der Kanülen-Modul (9) mit dem Befestigungsteil (24) auf dem Spritzenkörper (1) verrastet ist, wobei ein peripherer Ringwulst (7) am Spritzenkonus (6) in eine Ringnut (16) in der Innenwand des Befestigungsteils (24) eingreift.

19. Injektionsspritze nach Anspruch 18, dadurch gekennzeichnet, daß der Spritzenkonus (6) des Spritzenkörpers (1) an seinem hinteren Ende eine an den Ringwulst (7) anschließende periphere rundumlaufende und gegenüber dem Ringwulst (7) und vorzugsweise auch gegenüber dem Spritzenkonus (6) rückspringende Nut (8) aufweist, wobei der Kanülenschutz (10) unter Bildung einer an sich bekannten Schnapp- oder Rastverbindung korrespondierend hierzu ausgestaltet ist.

20. Injektionsspritze nach einem der Ansprüche 17 oder 19, dadurch gekennzeichnet, daß die Außenfläche des Spritzenkonus (6) zumindest teilweise aufgerauht ist.

21. Injektionsspritze nach einem der Ansprüche 17 bis 20, dadurch gekennzeichnet, daß der Spritzenkörper (1) aus einem Glaskörper besteht, dessen mit einem Gleitmittel versehene Innenwandung bei hoher Temperatur behandelt ist, vorteilhaft mit Silikon bei etwa 300°C.

22. Spritzenkörper (1) in einer Injektionsspritze nach Anspruch 17, mit einem Ansatz zur Befestigung eines Kanülen-Moduls (9) nach einem der Ansprüche 10 bis 16, dadurch gekennzeichnet, daß als Ansatz ein mit einem peripheren Ringwulst (7) versehener Spritzenkonus (6) vorgesehen ist.

23. Spritzenkörper (1) in einer Injektionsspritze nach Anspruch 17, mit einem Ansatz zur Befestigung eines Kanülen-Moduls (9) nach einem der Ansprüche 10 bis 16, dadurch gekennzeichnet, daß als Ansatz ein mit einer aufgerauhten Außenfläche versehener Spritzenkonus (6) vorgesehen ist.

24. Spritzenkörper nach Anspruch 22 oder 23, dadurch gekennzeichnet, daß der Spritzenkonus (6) an seinem hinteren Ende einen peripher umlaufenden Ringwulst (7) und eine an den Ringwulst (7) anschließende, peripher umlaufende, ringförmige Nut (8) aufweist.

25. Verfahren zum Montieren und Füllen einer Injektionsspritze nach einem der Ansprüche 17 bis 22, dadurch gekennzeichnet, daß der Spritzenkörper (1) gesondert innen silikonisiert und anschließend sterilisiert wird, daß unabhängig davon ein sterilisierter Kanülen-Modul (9) durch Einkleben der Kanüle (12) im Kanülenhalter (11), Einsetzen des Kanülenhalters (11) samt Kanüle (12) in den Kanülenschutz (10) sowie Aufsetzen der mit der stopfenförmigen Kanülendichtung (41) versehenen Stirnkappe (29) auf den Schutzhüllenteil (25), nach Prüfung auf Vorliegen einer Kanüle (12) in richtige Lage am offenen Ende des Schutzhüllenteils (25), zusammengebaut wird, und daß der Kanülen-Modul (9) unter sterilen Bedingungen auf den Spritzenkörper (1) dicht aufgesetzt und schließlich die Spritze befüllt sowie durch Einsetzen eines Kolbenstopfens (3) am hinteren Ende verschlossen wird.

26. Verfahren nach Anspruch 25, dadurch gekennzeichnet, daß die Teile des Kanülen-Moduls (9) vor dem Zusammenbau strahlensterilisiert werden.

## Claims

1. A cannula shield device (10) for an injection syringe, comprising a rear fastening portion (24) provided for attachment to a syringe body (1) and a forward sheath portion (25) integrally connected therewith and separable therefrom, for instance, via a predetermined breaking point (26), characterised in that a front cap (29) closed on its front side (30) and having an open rear end (31), which is provided as a separate component, is fastenable to the open forward end of the sheath portion (25) facing away from the fastening portion (24), and that a plug-like cannula seal (41) receiving the cannula tip (13) is received in the front cap (29).

2. A cannula shield device according to claim 1, characterised in that corresponding snap or latch connection elements (35, 35', 36) are molded on the sheath portion (25) and on the front cap (29), which are designed for placing the front cap (29) on the sheath portion (25).

3. A cannula shield device according to claim 1 or 2, characterised in that the sheath portion (25), in the region of its forward end, comprises a front-side abutment surface for the rear end (31) of the front cap (29).

4. A cannula shield device according to claim 3, characterised in that the front-side abutment surface of the sheath portion (25) is formed by the front face of a peripheral collar (38) integrally molded with the remaining sheath portion (25).

5. A cannula shield device according to any one of claims 1 to 4, characterised in that a forward predetermined breaking point (37) is provided on the sheath portion (25) adjacent the front end, yet at a distance from the site of attachment of the front cap (29).

6. A cannula shield device according to claim 4 and 5, characterised in that the collar (38) merges into a front cap carrying element (32) via the forward predetermined breaking point (37).

7. A cannula shield device according to any one of claims 2 to 6, characterised in that an annular latching groove (35) is formed in the forward end of the sheath portion (25), which groove cooperates with a radially inwardly protruding latch projection (35') provided on the rear end of the front cap (29), to undetachably fasten the front cap (29) to the sheath portion (25).

8. A cannula shield device according to any one of claims 1 to 7, characterised in that the fastening portion (24) comprises an inner cone (22) as well as an annular groove (20) for receiving a cannula holding device (11) equipped with a corresponding outer cone (21) and a corresponding peripherally extending annular projection.

9. A cannula shield device according to any one of claims 1 to 8, characterised in that a latching groove (16) is provided internally in the rear fastening portion (24), said latching groove matching with an annular bead (7) provided on a, for instance, conus-shaped hub (6) of the syringe body (1).

10. A cannula module (9) including a cannula shield device (10) according to any one of claims 1 to 9 and a cannula (12) received in a cannula holding device (11) within the interior of the cannula shield device, characterised in that the cannula (12), with its front end, protrudes beyond the open forward end of the sheath portion (25) and projects into the interior of the front cap (29), and that the cannula (12), on its rear end, is firmly secured in the cannula holding device (11), which, in turn, firmly fits in the rear zone of the cannula shield device (10).

11. A cannula module according to claim 10, characterised in that the cannula (12) is glued in the cannula holding device (11).

12. A cannula module according to claim 11, characterised in that the cannula holding device (11) comprises a conically widening adhesive take-up part (14) on its forward front end.

13. A cannula module according to any one of claims 10 to 12, characterised in that the cannula holding device (11) is rigidly inserted in the cannula shield device (10) surrounding the cannula (12), with the cannula holding device (11) having an outer cone (21) and the cannula shield device (10) having an inner cone (22) corresponding to the outer cone (21) of the cannula holding device (11).

14. A cannula module according to any one of claims 10 to 12, characterised in that the forward front side of the cannula holding device (11) is located slightly in front of the predetermined breaking point (26) enabling the separation of the sheath portion (25).

15. A cannula module according to any one of claims 10 to 14, characterised in that the cannula holding device (11) is formed from a rigid synthetic material, e.g. cellulose propionate, and that the cannula shield device (10) is formed from a softer synthetic material, e.g. polyethylene or polypropylene.

16. A cannula module according to any one of claims 13 to 15, characterised in that a peripherally extending annular projection (19) is provided on the outer cone (21) of the cannula holding device (11) on its end facing the syringe body (1), and that the cannula shield device (10), in its inner cone (22), has a groove (20) corresponding to this projection (19).

17. An injection syringe comprising a syringe body (1) and a cannula module (9) according to any one of claims 10 to 16, characterised in that the cannula module (9), with an arrangement of the cannula holding device (11) immediately forwardly of a syringe cone (6) provided on the syringe body (1), is put on this syringe cone (6) by the fastening portion (24) of its cannula shield device (10).

18. An injection syringe according to claim 17, characterised in that the cannula module (9) is latched with the fastening portion (24) on the syringe body (1), a peripheral annular bead (7) on the syringe cone (6) engaging in an annular groove (16) in the internal wall of the fastening portion (24).

19. An injection syringe according to claim 18, characterised in that the syringe cone (6) of the syringe body (1), in its rear end, includes a peripherally extending groove (8) following upon the annular bead (7) and re-entrant relative to the annular bead (7) and, preferably, also relative to the syringe cone (6), the cannula shield device (10) being designed in a corresponding manner under formation of a snap or latch connection known per se.

20. An injection syringe according to any one of claims 17 or 19, characterised in that the external surface of the syringe cone (6) is roughened, at least partially.

21. An injection syringe according to any one of claims 17 to 20, characterised in that the syringe body (1) consists of a glass body whose inner wall provided with a lubricant is treated at a high temperature, advantageously with silicone at approximately 300°C.

22. A syringe body (1) in an injection syringe according to claim 17, including a hub for attaching a cannula module (9) according to any one of claims 10 to 16, characterised in that a syringe cone (6) provided with a peripheral annular bead (7) is provided as said hub.

23. A syringe body (1) in an injection syringe according to claim 17, including a hub for attaching a cannula module (9) according to any one of claims 10 to 16, characterised in that a syringe cone (6) provided with a roughened external surface is provided as said hub.

24. A syringe body according to claim 22 or 23, characterised in that the syringe cone (6), on its rear end, comprises a peripherally extending annular bead (7) and a peripherally extending annular groove (8) following upon the annular bead (7).

25. A method of assembling and filling an injection syringe according to any one of claims 17 to 22, characterised in that the syringe body (1) internally is silicone-treated separately and subsequently is sterilized, that, independently thereof, a sterilized cannula module (9) is assembled by gluing the cannula (12) in the cannula holding device (11), inserting the cannula holding device (11) with the cannula (12) into the cannula shield device (10) and putting the front cap (29) provided with the plug-shaped cannula seal (41) on the sheath portion (25) after having checked the presence of a cannula (12) in the correct position at the open end of the sheath portion (25), and that the cannula module (9) is sealingly attached to the syringe body (1) under sterile conditions and, finally, the syringe is filled and closed on its rear end by inserting a plunger plug (3).

26. A method according to claim 25, characterised in that the elements of the cannula module (9) are sterilized by radiation before being assembled.

## Revendications

1. Embout protecteur de canule (10) pour une seringue d'injection, comportant une partie de fixation arrière (24) prévue pour la réunion avec une corps de seringue (1) et une partie formant manchon de protection (25), réunie d'une pièce, avec la partie de fixation et pouvant s'en séparer par l'intermédiaire d'une position de rupture préférentielle (26), caractérisé par le fait que sur l'extrémité avant ouverte, située du côté opposée à la partie de fixation (24), de la partie formant manchon de protection (25) peut se fixer un capuchon frontal (29), prévu comme pièce à part, fermé à sa face frontale avant (30), à l'extrémité arrière ouverte (31) et que dans le capuchon frontal (29) est logée une garniture d'étanchéité de la canule (41), en forme de bouchon, recevant la pointe de canule (13).

2. Embout protecteur de canule selon la revendication 1, caractérisé par le fait que sur la partie formant manchon de protection (25) et sur le capuchon frontal (29) sont formés des éléments d'encliquetage ou de crantage (35, 35', 36) conçus pour l'enfichage du capuchon frontal (29) sur la partie formant manchon de protection (25).

3. Embout protecteur de canule selon la revendication 1 ou 2, caractérisé par le fait que, dans la zone de son extrémité avant, la partie formant manchon de protection (25) présente une surface d'appui frontale pour l'extrémité arrière (31) du capuchon frontal (29).

4. Embout protecteur de canule selon la revendication 3, caractérisé par le fait que la surface d'appui frontale de la partie formant manchon de protection (25) est formée par la surface frontale d'un épaulement périphérique (38) formé, d'une pièce, avec le reste de la partie formant manchon de protection (25).

5. Embout protecteur de canule selon l'une des revendications 1 à 4, caractérisé par le fait que sur la partie formant manchon de protection (25), près de l'extrémité avant, mais à une certaine distance de la position de fixation du capuchon frontal (29), est prévue une position de rupture préférentielle (37).

6. Embout protecteur de canule selon la revendication 4 et 5, caractérisé par le fait que par l'intermédiaire de la position de rupture préférentielle avant (37), l'épaulement (38) se transforme en une partie (32) support du capuchon frontal.

7. Embout protecteur de canule selon l'une des revendications 2 à 6, caractérisé par le fait qu'à l'extrémité avant de la partie formant manchon de protection (25) est formée une rainure annulaire de crantage (35) qui collabore avec une saillie de crantage (35'), prévue à l'extrémité arrière du capuchon frontal (29), saillant radialement vers l'intérieur, pour la fixation imperdable du capuchon frontal (29) sur la partie formant manchon de protection (25).

8. Embout protecteur de canule selon l'une des revendications 1 à 7, caractérisé par le fait que la partie de fixation (24) présente un cône intérieur (22) ainsi qu'une rainure annulaire (20) pour recevoir un porte-canule (11) muni d'un cône extérieur correspondant (21) et d'une saillie annulaire périphérique correspondante.

9. Embout protecteur de canule selon l'une des revendications 1 à 8, caractérisé par le fait que sur la partie de fixation arrière (24), à l'intérieur, est prévue une rainure de crantage (16) qui s'ajuste sur un bourrelet annulaire (7) prévu sur un appendice (6), par exemple conique, du corps de seringue (1).

10. Module canule (9) comportant un embout protecteur de canule (10) selon l'une des revendications 1 à 9 et une canule (12) reçue dans un porte-canule (11), à l'intérieur de l'embout protecteur de canule, caractérisé par le fait que, côté frontal, la canule (12) dépasse au-delà de l'extrémité avant ouverte de la partie formant manchon de protection (25) et pénètre à l'intérieur du capuchon frontal (29), et qu'à son extrémité arrière, la canule (12) est fermement appliquée dans le porte-canule (11) qui, de son côté, a une assise ferme dans la zone inférieure de l'embout protecteur de canule (10).

11. Module canule selon la revendication 10, caractérisé par le fait que la canule (12) est collée dans le porte-canule (11).

12. Module canule selon la revendication 11, caractérisé par le fait qu'à l'extrémité frontale avant, le porte-canule (11) présente un réceptacle (14), qui s'élargit en cône, pour l'adhésif.

13. Module canule selon l'une des revendications 10 à 12, caractérisé par le fait que le porte-canule (11) est rigidement inséré dans l'embout protecteur de canule (10) qui entoure la canule (12), le porte-canule (11) présentant un cône extérieur (21) et l'embout protecteur de canule (10) présentant un cône intérieur (22) qui correspond au cône extérieur (21) du porte-canule (11).

14. Module canule selon l'une des revendications 10 à 12, caractérisé par le fait que la face frontale avant du porte-canule (11) vient un peu en avant de la position de rupture préférentielle (26) qui permet la séparation de la partie formant manchon de protection (25).

15. Module canule selon l'une des revendications 10 à 14, caractérisé par le fait que le porte-canule (11) est formé d'un matériau plastique dur, de préférence de propionate de cellulose, et que l' embout protecteur de canule (10) est formé d'un matériau plastique plus mou, par exemple de polyéthylène ou de polypropylène.

16. Module canule selon l'une des revendications 13 à 15, caractérisé par le fait que sur le cône extérieur (21) du porte-canule (11), à son extrémité située du côté du corps de seringue (1), est prévue une saillie annulaire périphérique (19) et que, sur son cône intérieur (22), l'embout protecteur de canule (10) présente une rainure (20) correspondant à cette saillie (19).

17. Seringue d'injection comportant un corps de seringue (1) et un module canule (9) selon l'une des revendications 10 à 16, caractérisée par le fait que, le porte-canule (11) venant immédiatement devant un cône de seringue (6) prévu sur le corps de seringue (1), le module canule (9) s'enfiche sur ce cône de seringue (6) par la partie de fixation (24) de son embout protecteur de canule (10).

18. Seringue d'injection selon la revendication 17, caractérisée par le fait que le module canule (9) se crante, par la partie de fixation (24) sur le corps de seringue (1), un bourrelet annulaire périphérique (7) prévu sur le cône de seringue (6), venant en prise dans une rainure annulaire (16) prévue dans la paroi intérieure de la partie de fixation (24).

19. Seringue d'injection selon la revendication 18, caractérisée par le fait qu'à son extrémité arrière, le cône de seringue (6) du corps de seringue (1) présente une rainure périphérique (8) qui jouxte le bourrelet annulaire (7) et qui est orientée vers l'arrière par rapport au bourrelet annulaire (7) et de préférence aussi par rapport au cône de seringue (6), l'embout protecteur de canule (10) étant réalisé de façon correspondante avec formation d'une liaison par encliquetage ou par crantage connue en soi.

20. Seringue d'injection selon l'une des revendication 17 à 19, caractérisée par le fait que la surface extérieure du cône de seringue (6) est, au moins partiellement, rendue rugueuse.

21. Seringue d'injection selon l'une des revendication 17 à 20, caractérisée par le fait que le corps de seringue (1) est constitué d'un corps vitreux dont la paroi intérieure, munie d'un lubrifiant, est traitée à haute température, de préférence avec de la silicone à environ 300°.

22. Corps de seringue (1) dans une seringue d'injection selon la revendication 17, comportant un appendice pour la fixation d'un module canule (9) selon l'une des revendications 10 à 16, caractérisé par le fait que comme appendice est prévu un cône de seringue (6) muni d'un bourrelet annulaire périphérique (7).

23. Corps de seringue (1) dans une seringue d'injection selon la revendication 17, comportant un appendice pour la fixation d'un module canule (9) selon l'une des revendications 10 à 16, caractérisé par le fait que comme appendice est prévu un cône de seringue (6) muni d'une surface extérieure rendue rugueuse.

24. Corps de seringue (1) selon l'une des revendications 22 ou 23, caractérisé par le fait qu'à son extrémité arrière le cône de seringue (6) présente un bourrelet annulaire périphérique (7) et une rainure annulaire périphérique (8) qui jouxte le bourrelet annulaire périphérique (7).

25. Procédé de montage et de remplissage d'une seringue d'injection selon l'une des revendications 17 à 22, caractérisé par le fait que l'on traite intérieurement, à part, à la silicone, le corps de seringue (1) et qu'ensuite on le stérilise, qu'indépendamment de cela, on assemble un module canule stérilisé (9) par collage de la canule (12) dans le porte-canule (11), insertion du porte-canule (11) avec la canule (12) dans l'embout protecteur de canule (10) et enfichage du capuchon frontal (29), muni de la garniture d'étanchéité de canule en forme de bouchon (41), sur la partie formant manchon de protection (25), après vérification de la présence d'une canule (12) en position correcte à l'extrémité ouverte de la partie formant manchon de protection (25), et qu'on enfiche de façon étanche le module canule (9) sur le corps de seringue (1) dans des conditions stériles et qu'enfin on remplit la seringue et qu'on la ferme par insertion, à l'extrémité arrière, d'un bouchon formant piston.

26. Procédé selon la revendication 25, caractérisé par le fait qu'avant l'assemblage on stérilise les parties du module canule (9) par des rayons.
